# EUROPÄISCHE PATENTSCHRIFT

(11) **EP 3 167 824 B1**
(45) Veröffentlichungstag und Bekanntmachung des Hinweises auf die Patenterteilung: **09.05.2018**
(21) Anmeldenummer: 16198516.3
(22) Anmeldetag: 11.11.2016
(51) Int. Cl.: A61B 17/29, A61B 18/14

(54) **DEMONTIERBARES CHIRURGISCHES INSTRUMENT**
SURGICAL INSTRUMENT WHICH CAN BE DISASSEMBLED
INSTRUMENT CHIRURGICAL DÉMONTABLE

(30) Priorität: 13.11.2015 DE 102015119691
(43) Veröffentlichungstag der Anmeldung: 17.05.2017
(73) Patentinhaber: KLS Martin GmbH + Co. KG, 79108 Freiburg (DE)
(72) Erfinder: Fritzsch, Gernod, 78532 Tuttlingen (DE); Herr, Sebastian, 79102 Freiburg i. Breisgau (DE)
(74) Vertreter: Maucher Jenkins Patent- und Rechtsanwälte

(56) Entgegenhaltungen:
- EP-B1- 2 246 004
- DE-A1- 3 716 764
- US-A- 5 290 309
- US-A- 5 873 873

## Beschreibung

### Gebiet der Erfindung

Die Erfindung betrifft ein chirurgisches Instrument umfassend einen Handgriff mit mindestens einem Betätigungselement, einen mit dem Handgriff verbundenen Rohrschaft mit einem ersten Rohr und einem, durch Betätigung des Betätigungselementes, entlang der Längsachse des Rohrschaftes relativ zu dem ersten Rohr beweglichen, zweiten Rohr und erste und zweite Backenelemente an dem distalen Ende des Rohrschafts, wobei das erste Backenelement fest und das zweite Backenelement über ein Gelenk beweglich mit dem Rohrschaft verbunden ist, wobei das zweite Rohr über eine Verbindungsstelle mit dem beweglichen Backenelement verbunden ist, so dass das bewegliche Backenelement durch Betätigung des Betätigungselements um das Gelenk schwenkbar ist.

### Hintergrund

Ein derartiges Instrument ist beispielsweise aus EP 2246004 B1 bekannt. Im Klinikbetrieb besteht der Bedarf nach einfach zu reinigenden, mehrfach verwendbaren chirurgischen Instrumenten. Diese sollen zudem möglichst betriebssicher und auch durch ungeübtes Personal zerlegbar sein. Das aus EP 2246004 B1 bekannte Instrument umfasst einen pistolenförmigen Handgriff und einen Rohrschaft mit mehreren, koaxial ineinander verlaufenden Rohren und einem Maulteil mit einem bewegliche Backenelement. Das beschriebene Instrument ist nicht zerlegbar. Es kann daher nicht innerlich gereinigt werden. Es ist lediglich für eine einzige Benutzung vorgesehen (sogenanntes "Single Use"-Instrument) und muss nach Benutzung aufwändig entsorgt werden.

Aus EP 2241273 A1 ist ein chirurgisches Rohrschaftinstrument bekannt, welches ein Außenrohr und ein Innenrohr umfasst, wobei das Innenrohr beweglich in dem Außenrohr gelagert ist und das Außenrohr eine Schneidvorrichtung aufweist. Die Schneidvorrichtung wird über das innere Rohr mittels einer Verzahnung angesteuert.

Aus EP 0797407 B1 ist ein chirurgisches Instrument mit einem Maulteil bekannt, wobei das Maulteil ebenfalls über ein inneres Rohr angelenkt wird und wobei das innere Rohr ein Widerlager aufweist, das über den Eingriff in eine entsprechende Aussparung des Maulteils axiale Kräfte übertragen kann.

Diese Instrumente bestehen allesamt aus vielen beweglichen Teilen in komplexen Ausgestaltungen. Sie haben daher den Nachteil, dass sie kompliziert zu demontieren und schwer zu reinigen sind.

Die Erfindung hat zur Aufgabe, ein vielseitig einsetzbares chirurgisches Instrument bereitzustellen, welches einfach zu demontieren und gut zu reinigen ist.

### Kurzbeschreibung der Erfindung

Diese Aufgabe wird durch die in den unabhängigen Ansprüchen angegebene Erfindung gelöst. Vorteilhafte Ausgestaltungen sind den Unteransprüchen zu entnehmen.
Erfindungsgemäß ist vorgesehen, dass die Verbindungsstelle und ein distaler Abschnitt des beweglichen Backenelementes, insbesondere das distale Ende des beweglichen Backenelementes auf einer Seite der Längsachse und das Gelenk auf der gegenüberliegenden Seite der Längsachse angeordnet sind, wobei die Verbindungsstelle durch einen Vorsprung an der auf der einen Seite der Längsachse liegenden Außenseite des beweglichen Backenelementes gebildet ist, der in eine Aussparung am distalen Ende des zweiten Rohres in Eingriff bringbar ist, und das zweite Rohr an seinem distalen Ende eine sich in axialer Richtung erstreckende Zunge aufweist, und die Aussparung in der Zunge angeordnet ist. Die Verbindungstelle und das bewegliche Backenelement können aus einem Stück gefertigt sein und somit ein einheitliches Bauteil bilden. Der Übergang vom Rohrschaft zum Maulteil ist daher ebenfalls sehr einfach gestaltbar. Auf weitere Öffnungen und Hinterschneidungen kann verzichtet werden. Zugleich kann die Verbindung zwischen dem Rohrschaft und dem Maulteil so ausgeführt werden, dass die Verbindungsstelle wenig exponiert und auf diese Weise vor Verschmutzung geschützt ist. Durch die Lage des Gelenkes in Bezug auf die Verbindungsstelle an dem beweglichen Backenelement ergibt sich eine Anordnung, die bei begrenztem Durchmesser des Instrumentes die maximale Hebelwirkung um den Drehpunkt erreicht und unter Einsatz weniger Teile hohe Schließkräfte und somit ein sicheres Ergreifen von Gewebe ermöglicht. sowie das Zerlegen des Rohrschaftes zur sicheren Reinigung ermöglicht.
Das Prinzip eines Eingriffes des Vorsprunges in eine Aussparung in einer Zunge ermöglicht eine Kraftübertragung mittels weniger Bauteile. Die Zunge des zweiten, beweglichen Rohres überdeckt in montierter Position die Verbindungsstelle und bewirkt, dass diese vor Verschmutzung geschützt ist. Das zweite Rohr ist relativ zu dem Handgriff und zu dem ersten Rohr beweglich. Das erste Rohr ist relativ zu dem Handgriff und zu dem zweiten Rohr feststehend. Zudem bildet das zweite, bewegliche Rohr eine einheitliche, zusammenhängende Oberfläche des Rohrschaftes, die nur wenige Durchbrechungen aufweist oder gar undurchbrochen ist. Dadurch werden Hinterschneidungen oder Kapillarspalte vermieden, in denen sich kontaminiertes Material anlagern kann. Durch die Anordnung der Aussparung in der Zunge ist diese ebenfalls leicht zugänglich und kann somit zuverlässig gereinigt werden. Das erfindungsgemäße Instrument ist zerlegbar. Es kann daher auch innerlich sicher gereinigt und desinfiziert und in Folge dessen mehrfach wiederverwendet werden. Beide Elemente sind zudem leicht herzustellen. Eine reduzierte Bauteilanzahl begünstigt die Langlebigkeit des Instrumentes.

In einer Ausgestaltung der Erfindung ist zwischen dem distalen Ende des zweiten, beweglichen Rohres und der Aussparung ein Steg gebildet, und an der Außenseite des beweglichen Backenelementes in distaler Richtung benachbart zu dem Vorsprung eine Vertiefung angeordnet, wobei der Steg in die Vertiefung eingreift oder darin zu sitzen kommt, wenn der Vorsprung an dem beweglichen Backenelement in die Aussparung in dem zweiten, beweglichen Rohr eingreift. Auf diese Weise ist eine einfache, lösbare mechanische Verbindung zwischen dem Rohrschaft und dem Maulteil geschaffen.

In einer Ausgestaltung der Erfindung weist das distale Ende des zweiten Rohres eine Stirnfläche auf, die bei einer distalen Bewegung des zweiten Rohres gegen einen weiteren Vorsprung an der Verbindungsstelle des beweglichen Backenelements anläuft und das Maulteil schließt.

Die durch die distale Bewegung des zweiten Rohres vermittelte Schließbewegung wird über die Stirnfläche des zweiten Rohres auf einen, am beweglichen Backenelement in distaler Richtung hinter der Vertiefung liegenden weiteren Vorsprung übertragen. Der weitere Vorsprung kann so ausgebildet sein, dass ein "Abrutschen" der Stirnfläche verhindert wird. Dazu können beispielsweise weitere Vertiefungen in der proximalen Flanke des weiteren Vorsprunges vorgesehen sein. Durch das axiale Anlaufen der Stirnfläche gegen den weiteren Vorsprung können starke Druckkräfte sicher in das bewegliche Backenelement eingeleitet werden.

In einer Ausgestaltung der Erfindung ist der Vorsprung in distaler Richtung ansteigend ausgebildet. Das distale Ende des beweglichen Rohres läuft dabei solange auf den rampen- oder keilförmigen Vorsprung auf, bis der Steg diesen übergreift und der Vorsprung in die Aussparung einrastet. Durch die ansteigende Form ist es möglich, den Steg des zweiten, Rohres in geschlossenem Zustand des Maulteils über den Vorsprung zu schieben.

In einer Ausgestaltung der Erfindung ist das erste Rohr innerhalb des zweiten Rohres angeordnet. Dies ermöglicht eine besonders platzsparende Bauweise und gewährleistet, dass das innere, feststehende Rohr weiterhin zur Durchführung von medizinischen Geräten, Sonden, Messern oder als Absaugkanal zur Verfügung steht, da es sich relativ zu durch das Rohr durchgeführten Geräten, Kabeln oder Leitungen nicht bewegt. Ferner ist es so möglich, im Innenraum des inneren, ersten Rohres befindliche Elemente abzuschirmen und vor äußeren Einflüssen zu schützen. Zudem kann diese Konstruktion eine einheitliche, glatte Oberfläche des Rohrschaftes begünstigen, die leicht einzuführen und gut zu reinigen ist.

In einer Ausgestaltung der Erfindung ist die Zunge flexibel oder elastisch ausgebildet. Beim Zusammenfügen des Instruments wird die Zunge über den oben beschriebenen rampenförmigen Vorsprung geführt und dabei radial nach außen gedrückt, bis der Vorsprung in die Aussparung in der Zunge eingreift und einrastet. Dies erleichtert die Montage.

In einer Ausgestaltung der Erfindung ist zwischen dem ersten und dem zweiten Rohr eine Begrenzungseinrichtung zum Begrenzen der axialen Bewegung des zweiten Rohres relativ zu dem ersten Rohr vorgesehen. Die Begrenzungseinrichtung definiert den Verschiebeweg der beiden Rohre relativ zueinander. Der Verschiebeweg legt den Öffnungswinkel der Backenelemente fest. Durch die so festgelegte Relativbewegung zwischen den Rohren werden die geöffnete und die geschlossene Position der Backenelemente festgelegt und begrenzt. Die Begrenzungseinrichtung ist dabei so ausgebildet, dass sie entlang der Längsachse Anschläge bildet. Die Anschläge verhindern, dass der festgelegte Bewegungsbereich bei der Bedienung überschritten werden kann. Versehentliche Fehlbedienungen werden so verhindert. Dies trägt wesentlich zur Handhabungssicherheit bei.

In einer Ausgestaltung der Erfindung umfasst die Begrenzungseinrichtung eine Aussparung und einen Vorsprung, wobei der Vorsprung entgegen einer Federkraft in der Aussparung gehalten und innerhalb der Aussparung entlang der Längsachse beweglich ist. Mit Vorteil ist die Aussparung in dem feststehenden Rohr als entlang der Längsachse verlaufende, beidseitig abgesetzte Nut ausgebildet. Dadurch bilden die Enden der Aussparung Anschläge für den Vorsprung. Die Relativbewegung wird dadurch sicher begrenzt. Der Vorsprung ist durch eine Feder vorgespannt. Die Federkraft presst den Vorsprung in die Aussparung und hält diesen in einer, die Relativbewegung der beiden Rohre begrenzenden Position. Die Vorspannung des Vorsprunges durch eine Feder sichert den Eingriff des Vorsprunges in die Aussparung und erhöht die Sicherheit gegen unbeabsichtigtes Lösen. Dies folgt dem "Fail-safe-Prinzip", ist konstruktiv einfach zu realisieren und bietet die erforderliche Betriebssicherheit.

In einer Ausgestaltung der Erfindung umfasst das Instrument einen Feststellmechanismus, wobei der Feststellmechanismus in einer ersten Stellung den Vorsprung innerhalb der Aussparung in radialer Richtung fixiert, und in einer zweiten Stellung ein radiales Verschieben des Vorsprunges gestattet, so dass der Vorsprung durch die Federkraft aus der Aussparung heraus beweglich ist. In der ersten Stellung greift der Vorsprung in die Aussparung ein und begrenzt die Relativbewegung der beiden Rohre wobei diese fest verbunden bleiben und nur um die Länge der Aussparung gegeneinander verschieblich sind. Die zweite Stellung ermöglicht das Lösen der beiden Rohre voneinander. Zur Demontage kann das erste, innere Rohr aus dem zweiten, äußeren Rohr herausgezogen werden. Dies ist von Vorteil, da eine Demontage entgegen der Flussrichtung einer kontaminierenden Flüssigkeit erfolgen kann und diese nicht durch Demontagetätigkeiten weiter ins Innere des Instrumentes befördert wird. Dies verbessert Hygiene und Langlebigkeit.

In einer Ausgestaltung der Erfindung umfasst der Feststellmechanismus einen Drehring, und ist durch Drehen des Drehringes von der ersten in die zweite Stellung überführbar.

Ein Drehring kann besonders leicht bedient werden, da er einen entsprechend großen Durchmesser aufweist. Der Drehring kann an seiner Außenseite Rutschmarkierungen aufweisen und somit einem Bediener anzeigen, ob Rohrschaft und Handgriff ordnungsgemäß und sicher verbunden sind. Die Anordnung ermöglicht auch den Verzicht auf einen kinematisch anspruchsvollen Mechanismus. Die wirkt sich günstig auf Anzahl und Struktur der umfassten Teile und somit auf die Reinigung aus. Eine Drehbewegung und damit das Lösen sind somit einfach möglich und kann auch mit Schutzhandschuhen von ungeübtem Personal durchgeführt werden.

In einer weiteren Ausgestaltung der Erfindung liegt der Vorsprung direkt oder indirekt einer Wand oder Oberfläche, insbesondere einer Innenseite des Drehrings an, wobei die Wand oder Oberfläche einen sich entlang des Umfanges verändernden radialen Abstand von der Längsachse aufweist, und wobei der Drehring durch Verdrehen relativ zu dem Vorsprung beweglich ist, so dass durch Verdrehen des Drehrings der radiale Abstand des Vorsprunges von der Längsachse veränderbar und der Vorsprung aus der Aussparung lösbar ist. Eine Drehbewegung und damit das Lösen und die Demontage des Rohrschaftes sind somit einfach und auch von Personal mit Schutzhandschuhen durchführbar.

In einer Ausgestaltung der Erfindung besitzt der Drehring eine elastische Zunge, die beim Betätigen einen federnd gelagerten Drücker radial bewegt und den Vorsprung aus der Aussparung drückt. Durch Drücken der elastischen Zunge durch einen Benutzer wird ein Drücker betätigt. Dadurch wird die Federkraft überwunden und der Vorsprung radial aus der Aussparung am ersten, inneren Rohr herausgedrückt. Dann ist das Zerlegen des Rohrschaftes möglich. Das Drücken der elastischen Zunge kann auch mit Schutzhandschuhen von ungeübtem Personal durchgeführt werden. Im Handgriff ist der der Rohrschaft mit nicht näher beschriebenen Rastmechanismen lösbar befestigt.

In einer Ausgestaltung ist das Instrument ein bipolares elektrochirurgisches Instrument umfassend eine erste und eine zweite Elektrode, wobei die erste Elektrode durch das bewegliche Backenelement gebildet ist, und die zweite Elektrode an oder auf dem festen Backenelement angeordnet und diesem gegenüber elektrisch isoliert ist. Das Instrument ist mit Vorteil als Kombiinstrument ausgebildet und erlaubt das Ergreifen, Koagulieren und/oder Trennen von Gewebe. Besonders günstig ist es, wenn das bewegliche Backenteil des Maulteils selbst die Elektroden bildet, da so auf eine separate Elektrode verzichtet werden kann.

In einer Ausgestaltung bildet das erste und/oder das zweite Rohr eine elektrische Verbindung zwischen der ersten Elektrode und einer Spannungsquelle. Auf diese Weise kann auf eine weitere stromführende Leitung in Form eines Kabels oder dgl. verzichtet werden. Dadurch wird eine Stromverbindung ohne ein zusätzliches, sich bewegendes Teil hergestellt, wodurch der Verschleiß verringert wird, was gerade bei einem wiederzuverwendenden Instrument von Bedeutung ist.

In einer Ausgestaltung ist die zweite Elektrode über ein nach außen hin elektrisch isoliertes Kabel mit der Spannungsquelle verbunden, wobei das Kabel in einer an der Außenseite des ersten Rohres gebildeten Nut zu dem Handgriff hin verläuft. Das Kabel ist in der Nut versenkt und darin fixiert. Vorzugsweise ist das Kabel in die Nut eingespritzt. Mit Vorteil ist die Nut mit dem Kabel mittels eines Füllstoffes ausgespritzt und schließt mit der äußeren Mantelfläche des Rohres flächenbündig ab. Auf diese Weise bleibt das Rohr gut zu reinigen und das Instrument kann entsprechend oft wiederverwendet werden.

In einer Ausgestaltung weist zumindest das äußere der beiden Rohre eine elektrisch isolierende Ummantelung auf. Die Isolierung dient zum Schutz des an dem äußeren Rohr anliegenden Gewebes bei der Behandlung.

In einer Ausgestaltung sind an dem Handgriff weitere Betätigungsmittel, insbesondere ein Taster oder eine Drücker, zum Schließen eines Stromkreises vorgesehen. Die Betätigungsmittel können simultan zu der Schließbewegung bedient werden können, so dass die Bedienung des Instruments erleichtert ist.

In einer Ausgestaltung ist die Verbindung zwischen dem beweglichen Backenelement und dem zweiten Rohr an der Verbindungsstelle lösbar, wobei auch die Verbindung zwischen dem Rohrschaft und dem Handgriff lösbar ist. Diese Ausgestaltung gestattet ein Zerlegen und Reinigen des Instruments. Somit ist das Instrument wiederverwendbar.

In einer Ausgestaltung ist das Betätigungselement um einen Drehpunkt schwenkbar gelagert, wobei der Drehpunkt auf derselben Seite der Längsachse angeordnet ist wie das Gelenk. Diese Ausgestaltung gestattet eine intuitive Betätigung des Instruments. Darüber hinaus ist diese Ausgestaltung hinsichtlich einer einfachen Zerlegung des Instruments von Vorteil.

### Kurzbeschreibung der Zeichnungen

Nachstehend sind anhand der Zeichnungen Ausführungsbeispiele der Erfindung näher beschrieben. Es zeigen:
Die Figur 1 eine beispielhafte Ausführungsform des erfindungsgemäßen chirurgischen Instruments;
die Figur 2 eine Schnittansicht des chirurgischen Instruments;
die Figur 3 eine beispielhafte Ausführungsform des Maulteils in Schnittdarstellung;
die Figur 4 eine beispielhafte Ausführungsform des erfindungsgemäßen Maulteils in perspektivischer Darstellung;
die Figur 5 ; eine beispielhafte Ausführungsform des distalen Endes des äußeren Rohres;
die Figur 6 eine beispielhafte Ausführungsform des proximalen Teils des Rohrschaftes in Schnittdarstellung
die Figur 7 eine beispielhafte Ausführungsform des beweglichen Backenelementes;
die Figur 8 eine weitere erfindungsgemäße Ausführungsform des Maulteils in perspektivischer Darstellung;
die Figur 9 die weitere erfindungsgemäße Ausführungsform des Maulteils aus Figur 8 in Schnittdarstellung;
die Figuren 10 - 12 ein Ausführungsbeispiel des erfindungsgemäßen Instrumentes mit geöffnetem, geschlossenem und überstrecktem Maulteil.

### Beschreibung der Erfindung anhand der beispielhaften, in den Zeichnungen beschriebenen Ausführungsformen

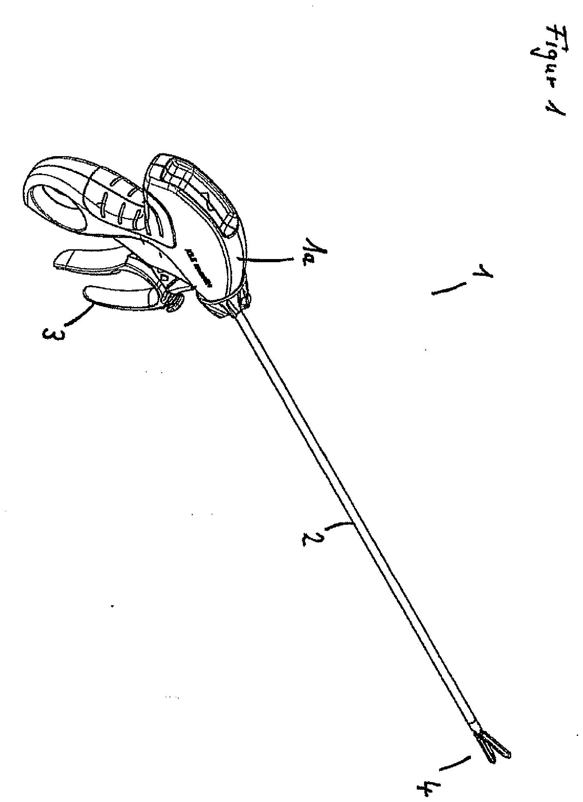

Figur 1 zeigt eine Ausführungsform des erfindungsgemäßen chirurgischen Instruments. Das Instrument umfasst einen pistolenförmigen Handgriff 1a, an welchem ein Rohrschaft 2 lösbar befestigt ist. Der Handgriff 1a umfasst einen Betätigungselement 3, mit welchem ein am distalen Ende des Rohrschaftes 2 angeordnetes Maulteil 4 betätigt, d.h. geöffnet und geschlossen werden kann. Ein derartiges Instrument ist beispielsweise in der sogenannten minimal invasiven Chirurgie einsetzbar.

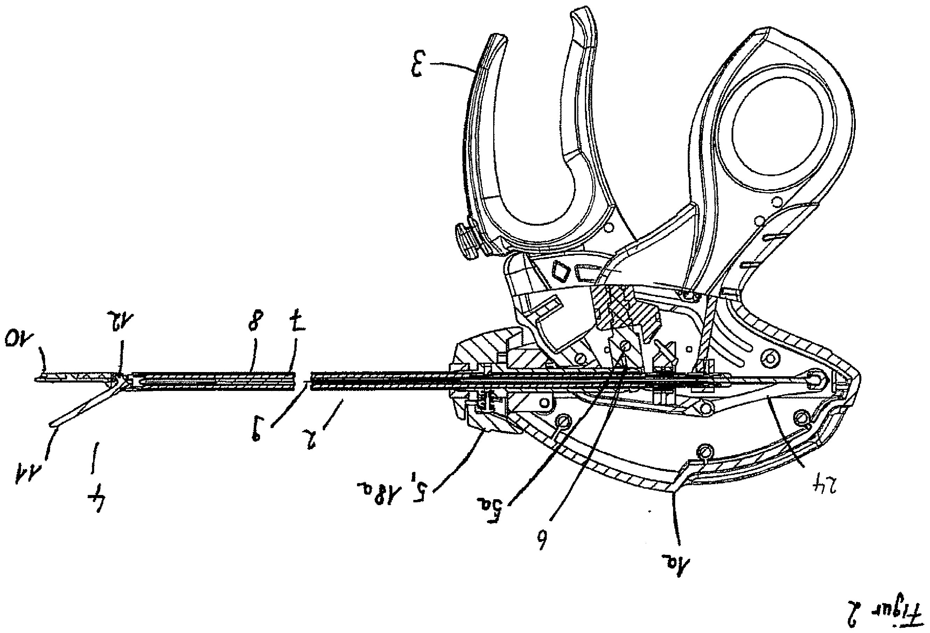

Figur 2 zeigt eine Schnittansicht des chirurgischen Instruments 1. Am handgriffseitigen Ende des Rohrschaftes ist ein Feststellmechanismus 5 angeordnet, der das Zerlegen des Rohrschaftes 2 ermöglicht.

Das Betätigungselement 3 ist in dem Handgriff 1a um eine Achse 5a schwenkbar gelagert und wirkt über eine Rastverbindung 6 mit dem Rohrschaft 2 zusammen. Die Achse 5a bildet einen Drehpunkt für das Betätigungselement 3 und ist unterhalb der Längsachse 9 angeordnet. Bei Betätigung des Betätigungselementes 3 wird das Maulteil 4 geschlossen. Dadurch sind die Bedienung und insbesondere das Ergreifen von Gewebe für den Bediener intuitiv durchführbar, da die Bewegung des Maulteils 4 der Handbewegung des Bedieners folgt. Der Rohrschaft 2 umfasst ein erstes inneres Rohr 7 und ein relativ dazu bewegliches zweites, äußeres Rohr 8. Das erste Rohr 7 ist in Bezug auf den Handgriff 1a feststehend. Die Rastverbindung 6 wirkt mit dem zweiten, äußeren Rohr 8 des Rohrschaftes 2 zusammen. Bei einer Betätigung des Betätigungselements 3 in proximaler Richtung wird das zweite, äußere Rohr 8 über die Rastverbindung 6 in distaler Richtung bewegt.

Der Rohrschaft 2 legt durch seine Erstreckung vom proximalen zum distalen Ende eine Längsachse 9 fest. Die Relativbewegung der beiden Rohre 7, 8 infolge einer Betätigung des Betätigungselements 3 erfolgt entlang der Längsachse 9.

Das am distalen Ende des Rohrschaftes 2 angeordnete Maulteil 4 umfasst ein feststehendes Backenelement 10 und ein relativ dazu bewegliches Backenelement 11. Das feststehende Backenelement 10 ist mit dem ersten, inneren Rohr 7 des Rohrschaftes 2 fest verbunden. Das bewegliche Backenelement 11 ist an dem feststehenden Backenelement 10 an einem Gelenk 12 drehbar angelagert.

Durch ein Lumen im ersten, inneren Rohr 7 kann ein weiteres Element z. Bsp. eine mechanisch wirkende Schneideinrichtung oder eine Laserfaser zum Maulteil 4 geführt und über ein separates Betätigungselement, das mit einem Schubgestänge 24 verbunden ist, in Längsrichtung bewegt werden.

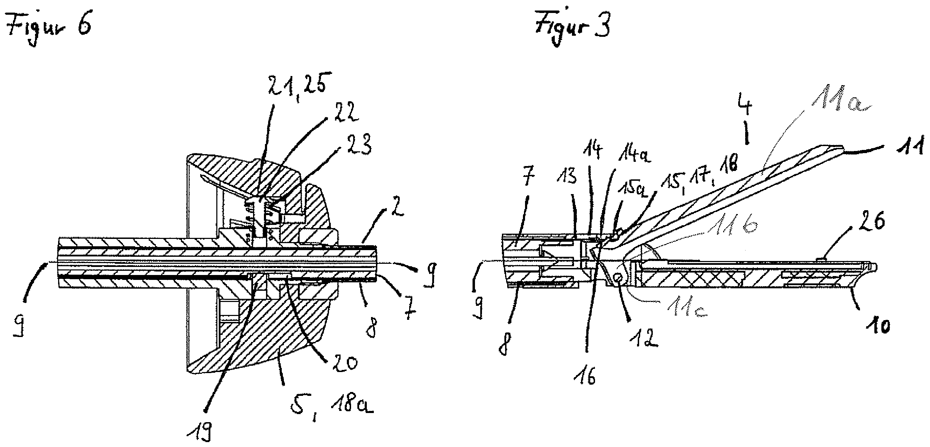

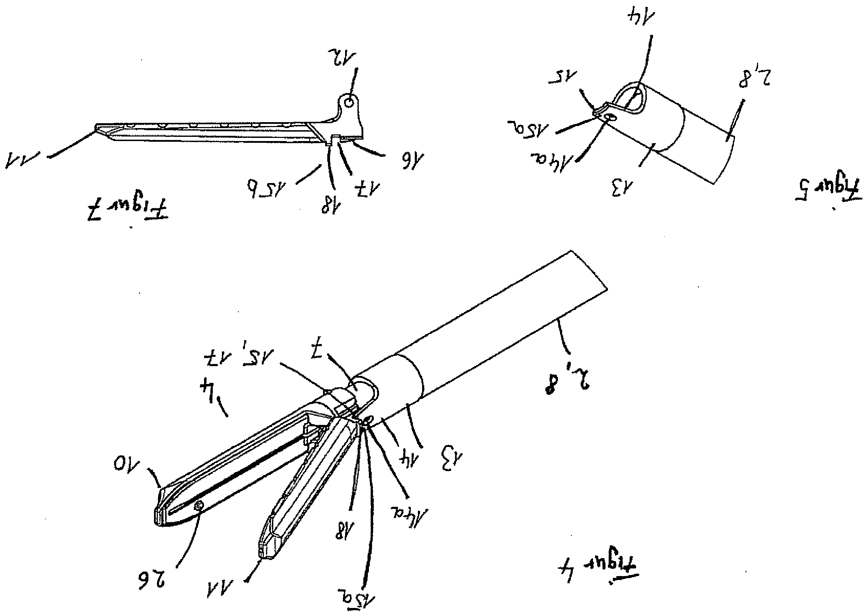

Die Figuren 3 und 4 zeigen Detailansichten des Maulteils 4 in geöffnetem Zustand. Das Gelenk 12 des beweglichen Backenelements 11 ist von der Längsachse 9 beabstandet angeordnet. In der dargestellten Orientierung befindet sich das Gelenk 12 unterhalb der Längsachse 9.

Das bewegliche Backenelement 11 umfasst einen distalen Abschnitt 11a und einen proximalen Abschnitt 11b. Der distale Abschnitt 11a umfasst das distale, freie Ende des Backenelementes 11. Am proximalen Abschnitt 11b ist ein Fortsatz 11c vorgesehen, der sich in einem Winkel, insbesondere eine rechten Winkel, zur Längserstreckung des Backenelementes 11 in Richtung zu dem feststehenden Backelement 10 (d.h. in der in Fig. 3 gezeigten Orientierung nach unten) hin erstreckt. Über den Fortsatz 11c ist das bewegliche Backenelement 11 schwenkbar an dem Gelenk 12 angelagert.

Wie beispielsweise in der Figur 3 dargestellt, erstreckt sich das bewegliche Backenelement 11, insbesondere der distale Abschnitt 11a und das distale Ende des Backenelementes 11 oberhalb der Längsachse 9, während sich zumindest das freie Ende des Fortsatzes 11c und das Gelenk 12 unterhalb der Längsachse 9 befinden.

Das zweite, äußere Rohr 8 weist an seinem distalen Ende einen Endabschnitt in Form einer Hülse 13 auf. Die Hülse 13 ist einstückig mit dem zweiten, äußeren Rohr 8 ausgeführt oder mit diesem fest verbunden. Die Hülse 13 weist oberhalb der Längsachse 9 eine Zunge 14 auf, über den das zweite, äußere Rohr 8 mit dem beweglichen Backenelement 11 verbunden ist. Die Zunge 14 weist eine Aussparung 14a auf, in die ein Vorsprung 16 am proximalen Ende des beweglichen Backenelementes 11 eingreift. Der Vorsprung 16 ist im Querschnitt keilförmig ausgebildet, d.h. er bildet eine in distaler Richtung ansteigende Rampe. Die Rampe erleichtert das Zusammenfügen des Rohrschafts 2, wie weiter unten beschrieben.

Durch die Aussparung 14a ist am distalen Ende der Zunge 14 ein Steg 15a gebildet (vgl. Fig. 5). Der Steg 15a greift in eine in distaler Richtung neben dem Vorsprung 16 angeordnete Vertiefung 17 an der Oberseite des Backenelements 11 ein. Auf diese Weise bilden die Aussparung 14a, der in die Aussparung 14a eingreifende Vorsprung 16, die Vertiefung 17 sowie der in die Vertiefung eingreifende Steg 15a einen Verbindungsbereich oder eine Verbindungsstelle 15b zwischen dem zweiten, äußeren Rohr 8 und dem oberen Backenelement 11. Die über die Verbindungsstelle 15b hergestellte Verbindung zwischen dem zweiten, äußeren Rohr 8 und dem oberen Backenelement 11 ist lösbar, wie weiter unten beschrieben.

Zum Öffnen des Maulteils 4 wird das zweite, äußere Rohr 8 in proximaler Richtung bewegt, wobei der Steg 15a in der Vertiefung 17 gegen die proximale Flanke der Vertiefung 17 anläuft und somit das obere Backenelement 11 um das Gelenk 12 herum - in der in Fig. 3 gezeigten Orientierung im Gegenuhrzeigersinn - schwenkt.

Umgekehrt wird das zweite, äußere Rohr 8 zum Schließen des Maulteils 4 in distaler Richtung bewegt, so dass der Steg 15a in der Vertiefung 17 gegen die distale Flanke der Vertiefung 17 anläuft und somit das obere Backenelement 11 um das Gelenk 12 herum - in der in Fig. 3 gezeigten Orientierung im Uhrzeigersinn - schwenkt.

Figur 5 zeigt eine Detailansicht des distalen Endes des zweiten, äußeren Rohres 8 umfassend die Hülse 13 mit der elastischen Zunge 14. Gut zu erkennen ist hier der durch den Bereich zwischen der Stirnseite 15 und der Aussparung 14a gebildete Steg 15a.

Beim Zusammenfügen des Rohrschafts 2 wird das erste, innere Rohr 7 in das zweite, äußere Rohr 8 hineingeschoben, bis das distale Ende der Zunge 14 auf den rampenartigen Vorsprung 16 trifft (vgl. Fig. 3 und 4). Durch weiteres Verschieben wird die elastische Zunge 14 durch die Rampe radial nach außen gedrückt, bis der Steg 15a am distalen Ende der Zunge 14 über den Rand der Rampe gleitet und aufgrund der Elastizität der Zunge 14, in die Vertiefung 17 schnappt. Gleichzeitig greift der Vorsprung 16 an der Oberseite des beweglichen Backenelementes 11 in die Aussparung 14a in der Zunge 14 ein.

Figur 6 zeigt eine Detaildarstellung des Feststellmechanismus 5.Der Feststellmechanismus 5 umfasst einen Drehring 18a, einen Drücker 22, eine Feder 23 und einem Vorsprung 19 in Form einer Rastnase. Der Feststellmechanismus 5 dient zur axialen Begrenzung der Relativbewegung der beiden Rohre 7, 8. Der Vorsprung 19 ist in der dargestellten Orientierung unterhalb der Längsachse 9 angeordnet und innerhalb des Drehringes 18a federkraftbeaufschlagt. Im eingerasteten Zustand greift der Vorsprung 19 in eine Aussparung 20 des ersten, inneren Rohres 7 ein. In einer nicht dargestellten Ausführungsform ist der Rohrschaft 2 mittels des Drehringes 18a um die Längsachse 9 rotierbar.

Der Feststellmechanismus 5 umfasst weiterhin einen entlang des Umfanges ansteigenden Vorsprung 21. Insbesondere ist der ansteigende Vorsprung 21 durch eine in Umfangsrichtung zulaufende Rampe an einer Innenseite des Drehringes 18a gebildet und befindet sich oberhalb der Längsachse in Flucht des Vorsprunges 19. Bei einer Drehung des Drehringes 18a wirkt der Vorsprung 21 mit einem federnd gelagerten Drücker 22 zusammen, welcher die Bewegung des Drehringes 18a auf den Vorsprung 19 überträgt. Durch die Drehung des Drehringes 18a läuft der rampenförmige Vorsprung 21 auf den Drücker 22 auf und übt auf diesen eine Druckkraft entgegen der Federspannung aus. Der Vorsprung 19 wird durch den Drücker 22 radial von der Längsachse 9 wegbewegt und gelangt mit der Aussparung 20 des inneren Rohres 7 außer Eingriff. In einer weiteren Ausgestaltung der Erfindung ist der Drehring 18a mit einer elastischen Zunge 25 versehen, die beim Betätigen den federnd gelagerten Drücker 22 auf die Längsachse 9 des Rohrschaftes 2 zu bewegt und den Vorsprung 19 in gleicher Weise mit der Aussparung 20 außer Eingriff bringt.

In dem in Fig. 6 dargestellten eingerasteten Zustand befindet sich der Vorsprung 19 in der Aussparung 20 und begrenzt die axiale Relativbewegung des zweiten, äußeren Rohres 8 in Bezug auf das erste, innere Rohr 7.

Figur 7 zeigt das bewegliche Backenelement 11. Gut zu erkennen ist hier die Keilform des Vorsprungs 16. Wie oben beschrieben gleitet der Steg 15a der elastischen Zunge 14 beim distalen Verschieben des zweiten, äußeren Rohres 8 über den keilförmigen Vorsprung 16 bis in die Vertiefung 17. Dabei rastet der Vorsprung 16 in die Aussparung 14 der Hülse 13 ein. Bei der Schwenkbewegung des beweglichen Backenelementes 11, die durch Verschieben des zweiten, äußeren Rohres 8 entlang der Längsachse 9 erfolgt, gelangt der in distaler Richtung hinter der Aussparung 14a liegende Steg 15a mit der Vertiefung 17 in Eingriff. Die Stirnfläche 15 und der Steg 15a tauchen beim Öffnen des Maulteils 4 in die Vertiefung 17 ein. Die Vertiefung 17 ist so dimensioniert, dass bei einer distalen Bewegung des zweiten, äußeren Rohres 8 die Stirnfläche 15 die Schubkraft auf den weiteren Vorsprung 18 am beweglichen Backenelement 11 überträgt und das Maulteil 4 schließt.

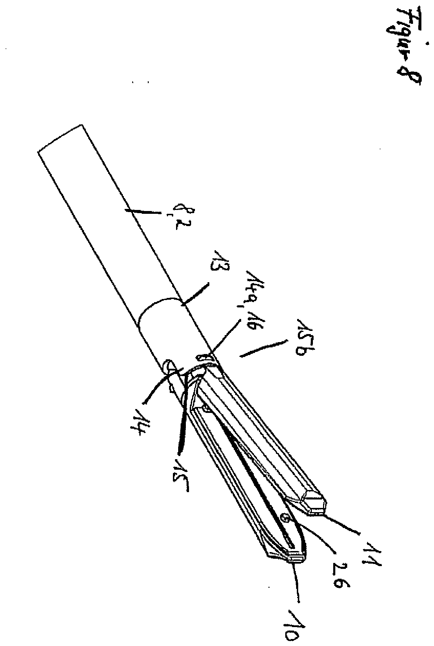

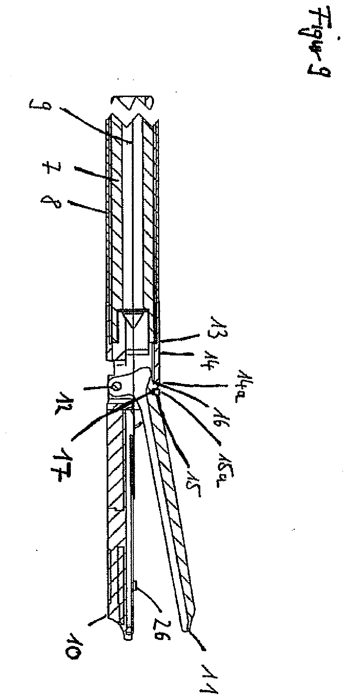

Die Figuren 8 und 9 zeigen einen Ausschnitt des Instruments 1 gemäß einer anderen Ausgestaltung der Erfindung. Diese Ausgestaltung unterscheidet sich von der Ausgestaltung gemäß den Figuren 1-7 darin, dass der Vorsprung 16 nicht rampen- oder keilförmig ausgebildet ist, sondern im Querschnitt die Form eines Rechteckes oder Trapezes aufweist. Der Vorsprung 16 ist als einzeln stehender Vorsprung ausgebildet. In dieser Ausgestaltungsform wird auf den weiteren Vorsprung 18 verzichtet. In dieser Ausgestaltungsform werden die Kräfte zum Öffnen und Schließen des Maulteils allein über die proximale und die distale Seite des Vorsprunges 16 auf das bewegliche Backenelement 11 übertragen. Befindet sich das Instrument in montiertem, gebrauchsbereitem Zustand, übergreift die Zunge 14 der Hülse 13 den Vorsprung 16, so dass die Aussparung 14a mit dem Vorsprung 16 in Eingriff steht. Zum Aufschwenken des beweglichen Backenelementes 11 nimmt der distale Teil des Vorsprungs 16 die durch die distale Innenseite der Aussparung 14a übertragene Zugkraft auf.

Beim Schließen des Maulteils bewegt sich das zweite, äußere Rohr 8 in distaler Richtung. Die aus dieser Bewegung herrührende Schließkraft wird über die proximale Innenseite der Aussparung 14a auf die proximale Seite des Vorsprungs 16 übertragen. Diese Übertragung ist möglich, weil der Vorsprung 16 nicht rampen- oder keilförmig und in distaler Richtung ansteigend ausgebildet ist. Die proximale Innenseite der Aussparung 14a und der proximale Teil des Vorsprunges 16 bilden bei geschlossenem Maulteil 4 parallele Flächen.

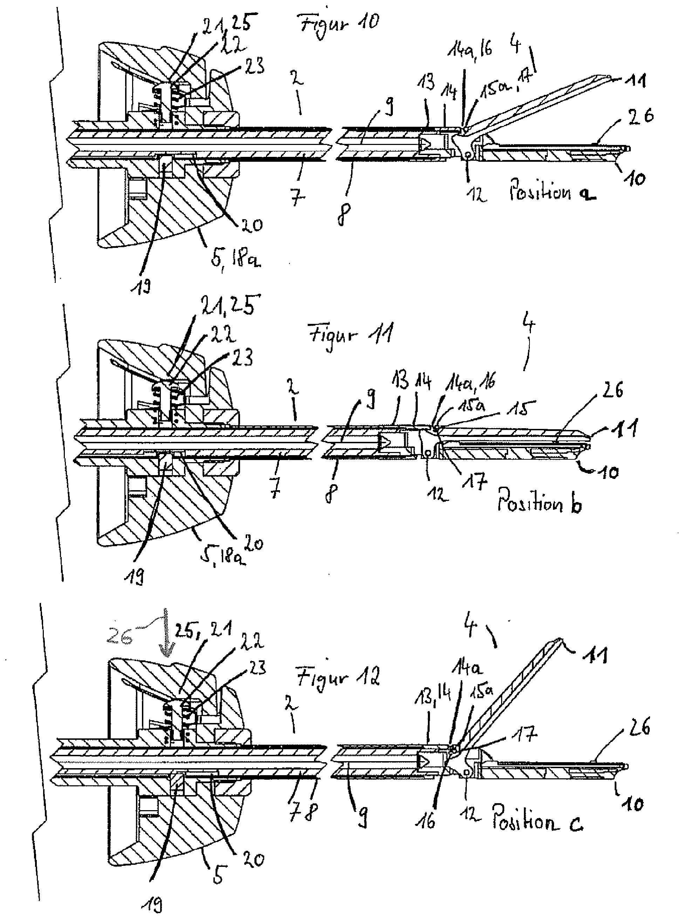

Die Figuren 10 bis 12 zeigen im Querschnitt drei Betriebszustände des Instruments 1 in der zweiten beschriebenen Ausführungsform der Verbindungsstelle 15b mit einzeln stehendem Vorsprung 16, gemäß den Figuren 8 und 9. Figur 10 zeigt das Maulteil 4 in geöffnetem Zustand. Die Haltekräfte für das bewegliche Backenelement 11 werden dabei wie unter Figur 9 beschrieben übertragen. Der Vorsprung 19 steht mit der Aussparung 20 am inneren Rohr 7 in Eingriff und begrenzt die Bewegung des äußeren Rohres 8 in proximaler Richtung. Die Begrenzung erfolgt dabei durch Anschlag des Vorsprunges 19 an der proximalen Kante der als abgesetzte Nut ausgebildeten Aussparung 20. Die Abmessung der Aussparung 20 legt den Bewegungsbereich des Vorsprunges 19 fest. Damit wird der Bewegungsbereich des äußeren Rohres 8 und der Schwenkbereich des beweglichen Backenelementes 11 festgelegt. In dem dargestellten geöffneten Gebrauchszustand liegt der Vorsprung 19 am proximalen Ende der Aussparung 20 an. Dies ist für einen Benutzer bei der Betätigung des Betätigungselementes 3 als Widerstand haptisch wahrnehmbar.

Die Figur 11 zeigt das Maulteil 4 in geschlossenem Zustand. Das äußere Rohr 8 ist dabei in distaler Richtung verschoben. Der Vorsprung 19 steht mit der Aussparung 20 in Eingriff. Die distale Verschiebebewegung des zweiten, äußeren Rohres 8 wird durch das Aufliegen des beweglichen Backenelementes auf einen Abstandshalter 26 der sich auf dem feststehenden Backenelement 10 des Maulteils 4 befindet begrenzt. Die proximale Innenseite der Aussparung 14a überträgt die Schließkraft auf die proximale Seite des Vorsprunges 16. Beide liegen in dieser Stellung etwa parallel zueinander. Der Steg 15a liegt oberhalb der Vertiefung 17. In geschlossenem Zustand liegen auch die Innenflächen der Backenelemente 10,11 parallel zueinander.

In Figur 12 befindet sich das Maulteil 4 in einer überstreckten Stellung. Überstreckt bedeutet, dass das bewegliche Backenelement 11 in Bezug auf das feststehende Backenelement 10 über einen, durch die Begrenzungseinrichtung festgelegten Schwenkbereich hinaus aufgeschwenkt ist. Durch eine Drehbewegung des Drehringes 18a oder durch Drücken der elastischen Zunge 25 am Drehring 18a gelangt der Vorsprung 19 mit der Aussparung 20 außer Eingriff, so dass das zweite, äußere Rohr so weit in proximaler Richtung bewegt werden kann, dass der Vorsprung 16 sich aus der Aussparung 14a löst. Der Vorsprung 19 ist über die proximale Kante der Aussparung 20 bewegt. Das Bewegen des Vorsprunges 19 über die proximale Kante der Aussparung 20 ist vom Bediener durch einen Widerstand bei der Bewegung des Betätigungselementes 3 wahrnehmbar. Durch "Überdrücken" des Widerstandes bewegt sich der Vorsprung 16 dann über die Aussparung 20 in proximaler Richtung hinaus und gibt die Relativbewegung der beiden Rohre frei.

Eine weitere Möglichkeit der Betätigung besteht darin, dass im Drehrad ein Abschnitt als elastische Zunge 25 ausgebildet ist. Durch radialen Druck auf diese Zunge, entsprechend dem Richtungspfeil 26, wird der Drücker 22 entgegen der Kraft der Feder 23 radial in Richtung der Längsachse des Rohrschaftes 9 bewegt und der mit dem Drücker verbundene Vorsprung 19 bewegt sich simultan so weit von der Längsachse weg, dass er die proximale Kante der Aussparung 20 überwindet und die Relativbewegung der beiden Rohre frei gibt.

Das erste, innere Rohr 7 kann dann vollständig aus dem zweiten, äußeren Rohr 8 herausgezogen werden. Bei der Montage wird das bewegliche Backenelement 11 in die überstreckt geöffnete Position c geschwenkt. Die Aussparung 14a in der Hülse 13 wird über den Vorsprung 16 geschoben und das Maulteil 4 geschlossen, so dass der Vorsprung 16 in die Aussparung 14 einrastet. Der Demontagevorgang ist somit einfach durchführbar und umfasst die drei Schritte proximales Verschieben des zweiten, äußeren Rohres 8 nach Betätigung des Drehringes 18a, Ausrasten des Vorsprunges 16 aus der Aussparung 14a und Herausziehen des ersten, inneren Rohres 7 aus zweiten, äußeren Rohr 8. Der Montagevorgang erfolgt in umgekehrter Reihenfolge.

### Bezugszeichenliste

- 1: Chirurgisches Instrument
- 1a: Handgriff
- 2: Rohrschaft
- 3: Betätigungselement
- 4: Maulteil
- 5: Feststellmechanismus
- 5a: Achse des Betätigungselementes
- 6: Rastverbindung
- 7: Inneres Rohr/ erstes Rohr
- 8: Äußeres Rohr/ zweites Rohr
- 9: Längsachse (des Rohrschaftes)
- 10: feststehendes Backenelement
- 11: bewegliches Backenelement
- 11a: distaler Abschnitt
- 11b: proximaler Abschnitt
- 11c: Fortsatz
- 12: Gelenk
- 13: Hülse
- 14: Zunge
- 14a: Aussparung (in der Hülse)
- 15: Stirnfläche
- 15a: Steg
- 15b: Verbindungsstelle
- 16: Vorsprung
- 17: Vertiefung
- 18: weiterer Vorsprung
- 18a: Drehring
- 19: Vorsprung
- 20: Aussparung am inneren Rohr
- 21: ansteigender Vorsprung (im Drehring)
- 22: Drücker
- 23: Feder
- 24: Schubgestänge
- 25: elastische Zunge
- 26: Abstandhalter
- 27: Richtungspfeil

## Patentansprüche

1. Chirurgisches Instrument umfassend:
einen Handgriff (1a) mit mindestens einem Betätigungselement (3);
einen mit dem Handgriff verbundenen Rohrschaft (2) mit einem ersten Rohr (7) und einem, durch Betätigung des Betätigungselementes (3), entlang der Längsachse (9) des Rohrschaftes (2) relativ zu dem ersten Rohr (7) beweglichen zweiten Rohr (8); und
erste und zweite Backenelemente (10,11) an dem distalen Ende des Rohrschafts (2), wobei das erste Backenelement(10) fest und das zweite Backenelement (11) über ein Gelenk (12) beweglich mit dem Rohrschaft (2) verbunden ist;
wobei das zweite Rohr (8) über eine Verbindungsstelle (15b) mit dem beweglichen Backenelement (11) verbunden ist, so dass das bewegliche Backenelement (11) durch Betätigung des Betätigungselements (3) um das Gelenk (12) schwenkbar ist;
**dadurch gekennzeichnet, dass** die Verbindungsstelle (15b) und ein distaler Abschnitt (11a) des beweglichen Backenelementes (11), insbesondere das distale Ende des beweglichen Backenelementes (11) auf einer Seite der Längsachse (9) und das Gelenk (12) auf der gegenüberliegenden Seite der Längsachse (9) angeordnet sind, wobei die Verbindungsstelle (15b) durch einen Vorsprung (16) an der auf der einen Seite der Längsachse (9) liegenden Außenseite des beweglichen Backenelementes (11) gebildet ist, der in eine Aussparung (14a) am distalen Ende des zweiten Rohres (8) in Eingriff bringbar ist, und das zweite Rohr (8) an seinem distalen Ende eine sich in axialer Richtung erstreckende Zunge (14) aufweist, und die Aussparung (14a) in der Zunge (14) angeordnet ist.

2. Chirurgisches Instrument nach Anspruch 1, **dadurch gekennzeichnet, dass** zwischen dem distalen Ende des zweiten Rohres (8) und der Aussparung (14a) ein Steg (15a) gebildet ist, und dass an der Außenseite des beweglichen Backenelementes (11) in distaler Richtung benachbart zu dem Vorsprung (16) eine Vertiefung (17) angeordnet ist, wobei der Steg (15a) in die Vertiefung (17) eingreift oder darin zu sitzen kommt, wenn der Vorsprung (16) an dem beweglichen Backenelement (11) in die Aussparung (14a) in dem beweglichen Rohr eingreift.

3. Chirurgisches Instrument nach einem der vorhergehenden Ansprüche, **dadurch gekennzeichnet, dass** das distale Ende des zweiten Rohres (8) eine Stirnfläche (15) aufweist, die bei einer distalen Bewegung des zweiten Rohres (8) gegen einen weiteren Vorsprung (18) an der Verbindungsstelle (15a) des beweglichen Backenelements (11) anläuft und das Maulteil 4 schließt.

4. Chirurgisches Instrument nach einem der vorhergehenden Ansprüche, **dadurch gekennzeichnet, dass** der Vorsprung (16) in distaler Richtung ansteigend ausgebildet ist und/oder die Zunge flexibel oder elastisch ausgebildet ist.

5. Chirurgisches Instrument nach einem der vorhergehenden Ansprüche, **dadurch gekennzeichnet, dass** das erste Rohr (7) innerhalb des zweiten Rohres (8) angeordnet ist.

6. Chirurgisches Instrument nach einem der vorhergehenden Ansprüche, mit einer Begrenzungseinrichtung zwischen dem ersten und dem zweiten Rohr (7,8) zum Begrenzen der axialen Bewegung des zweiten Rohres (8) relativ zu dem ersten Rohr (7), wobei insbesondere die Begrenzungseinrichtung eine Aussparung (20) und einen Vorsprung (19) umfasst, wobei der Vorsprung (19) entgegen einer Federkraft in der Aussparung gehalten und innerhalb der Aussparung entlang der Längsachse (9) beweglich ist.

7. Chirurgisches Instrument nach Anspruch 6, mit einem Feststellmechanismus (5), wobei der Feststellmechanismus (5) in einer ersten Stellung den Vorsprung (19) innerhalb der Aussparung (20) in radialer Richtung fixiert, und in einer zweiten Stellung ein radiales Verschieben des Vorsprunges (19) gestattet, so dass der Vorsprung (19) entgegen der Federkraft aus der Aussparung (20) heraus beweglich ist.

8. Chirurgisches Instrument nach Anspruch 7, wobei der Feststellmechanismus (5) einen Drehring (18a) umfasst, und der Feststellmechanismus durch Drehen des Drehringes von der ersten in die zweite Stellung überführbar ist, und insbesondere wobei der Vorsprung (19) direkt oder indirekt an einer Wand oder Oberfläche, insbesondere einer Innenseite des Drehrings anliegt, wobei die Wand oder Oberfläche einen sich entlang des Umfanges verändernden radialen Abstand von der Längsachse aufweist, und wobei der Drehring (18a) durch Verdrehen relativ zu dem Vorsprung beweglich ist, so dass durch Verdrehen des Drehrings der radiale Abstand des Vorsprunges von der Längsachse veränderbar und der Vorsprung aus der Aussparung lösbar ist, oder wobei der Feststellmechanismus (5) eine elastische Zunge (25) aufweist, die beim Betätigen einen federnd gelagerten Drücker (22) radial bewegt und den Vorsprung (19) aus der Aussparung (20) drückt.

9. Chirurgisches Instrument (1) nach einem der vorhergehenden Ansprüche wobei das Instrument (1) ein bipolares elektrochirurgisches Instrument umfassend eine erste und eine zweite Elektrode ist, wobei die erste Elektrode durch das bewegliche Backenelement (11) gebildet ist, und die zweite Elektrode an oder auf dem festen Backenelement angeordnet und diesem gegenüber elektrisch isoliert ist.

10. Chirurgisches Instrument (1) nach Anspruch 9, **dadurch gekennzeichnet, dass** das erste und/oder das zweite Rohr eine elektrische Verbindung zwischen der ersten Elektrode und einer Spannungsquelle bildet.

11. Chirurgisches Instrument (1) nach Anspruch 9 oder 10, **dadurch gekennzeichnet, dass** die zweite Elektrode über ein nach außen hin elektrisch isoliertes Kabel mit der Spannungsquelle verbunden ist, wobei das Kabel in einer an der Außenseite des ersten Rohres (7) gebildeten Nut zu dem Handgriff (1a) hin verläuft.

12. Chirurgisches Instrument (1) nach einem der vorhergehenden Ansprüche, wobei zumindest das äußere der beiden Rohre (7,8) eine elektrisch isolierende Ummantelung aufweist.

13. Chirurgisches Instrument nach einem der vorhergehenden Ansprüche, **dadurch gekennzeichnet, dass** an dem Handgriff (1a) weitere Betätigungsmittel, insbesondere ein Taster oder eine Drücker, zum Schließen eines Stromkreises vorgesehen sind.

14. Chirurgisches Instrument nach einem der vorhergehenden Ansprüche, wobei die Verbindung zwischen dem beweglichen Backenelement (11) und dem zweiten Rohr (8) an der Verbindungsstelle (15b) lösbar ist, und wobei die Verbindung zwischen dem Rohrschaft und dem Handgriff lösbar ist.

15. Chirurgisches Instrument nach einem der vorhergehenden Ansprüche, wobei das Betätigungselement (3) um einen Drehpunkt (5a) schwenkbar gelagert ist, wobei der Drehpunkt (5a) auf derselben Seite der Längsachse (9) angeordnet ist wie das Gelenk (12).

## Claims

1. Surgical instrument comprising:
a handle (1a) with at least one actuating element (3) ;
a tubular shaft (2) connected to the handle and provided with a first tube (7) and with a second tube (8) movable relative to the first tube (7) along the longitudinal axis (9) of the tubular shaft (2) by actuation of the actuating element (3); and
first and second jaw elements (10, 11) at the distal end of the tubular shaft (2), wherein the first jaw element (10) is connected rigidly to the tubular shaft (2) and the second jaw element (11) is connected movably to the tubular shaft (2) via a joint (12) ;
wherein the second tube (8) is connected to the movable jaw element (11) via a connection site (15b), such that the movable jaw element (11) is pivotable about the joint (12) by actuation of the actuating element (3);
**characterized in that** the connection site (15b) and a distal portion (11a) of the movable jaw element (11), in particular the distal end of the movable jaw element (11), are arranged on one side of the longitudinal axis (9) and the joint (12) is arranged on the opposite side of the longitudinal axis (9), wherein the connection site (15b) is formed by a projection (16) on the outside of the movable jaw element (11) on one side of the longitudinal axis (9), which projection (16) can be brought into engagement in a recess (14a) at the distal end of the second tube (8), and the second tube (8) has, at its distal end, an axially extending tongue (14), and the recess (14a) is arranged in the tongue (14).

2. Surgical instrument according to Claim 1, **characterized in that** a web (15a) is formed between the distal end of the second tube (8) and the recess (14a), and **in that** a depression (17) is arranged on the outside of the movable jaw element (11), distally adjacent to the projection (16), wherein the web (15a) engages in the depression (17) or comes to sit in the latter when the projection (16) on the movable jaw element (11) engages in the recess (14a) in the movable tube.

3. Surgical instrument according to one of the preceding claims, **characterized in that** the distal end of the second tube (8) has a front face (15) which, upon a distal movement of the second tube (8), runs up against a further projection (18) at the connection site (15a) of the movable jaw element (11) and closes the mouth part (4).

4. Surgical instrument according to one of the preceding claims, **characterized in that** the projection (16) is designed ascending in the distal direction, and/or the tongue is flexible or elastic.

5. Surgical instrument according to one of the preceding claims, **characterized in that** the first tube (7) is arranged inside the second tube (8).

6. Surgical instrument according to one of the preceding claims, with a limit mechanism between the first and second tubes (7, 8) for limiting the axial movement of the second tube (8) relative to the first tube (7), wherein in particular the limit mechanism comprises a recess (20) and a projection (19), wherein the projection (19) is held in the recess counter to a spring force and is movable inside the recess along the longitudinal axis (9).

7. Surgical instrument according to Claim 6, with a securing mechanism (5), wherein the securing mechanism (5), in a first position, fixes the projection (19) in the radial direction inside the recess (20) and, in a second position, permits a radial displacement of the projection (19), such that the projection (19) can be moved out of the recess (20) counter to the spring force.

8. Surgical instrument according to Claim 7, wherein the securing mechanism (5) comprises a rotary ring (18a), and the securing mechanism can be transferred from the first position to the second position by rotation of the rotary ring, and in particular wherein the projection (19) bears directly or indirectly on a wall or surface, in particular an inner face of the rotary ring, wherein the wall or surface is spaced radially apart from the longitudinal axis by a distance that varies along the circumference, and wherein the rotary ring (18a) is movable relative to the projection by rotation, such that the radial distance of the projection from the longitudinal axis can be modified by rotation of the rotary ring and the projection can be released from the recess, or wherein the securing mechanism (5) has an elastic tongue (25) which, upon actuation, moves a spring-mounted pusher (22) radially and pushes the projection (19) out of the recess (20).

9. Surgical instrument (1) according to one of the preceding claims, wherein the instrument (1) is a bipolar electrosurgical instrument comprising a first electrode and a second electrode, wherein the first electrode is formed by the movable jaw element (11), and the second electrode is arranged at or on the rigid jaw element and is electrically insulated from the latter.

10. Surgical instrument (1) according to Claim 9, **characterized in that** the first tube and/or the second tube forms an electrical connection between the first electrode and a voltage source.

11. Surgical instrument (1) according to Claim 9 or 10, **characterized in that** the second electrode is connected to the voltage source by a cable electrically insulated from the outside, wherein the cable runs towards the handle (1a) in a groove formed on the outside of the first tube (7).

12. Surgical instrument (1) according to one of the preceding claims, wherein at least the outer of the two tubes (7, 8) has an electrically insulating sheath.

13. Surgical instrument according to one of the preceding claims, **characterized in that** further actuating means, in particular a pushbutton or a slide, are provided on the handle (1a) for the purpose of closing a current circuit.

14. Surgical instrument according to one of the preceding claims, wherein the connection between the movable jaw element (11) and the second tube (8) is releasable at the connection site (15b), and wherein the connection between the tubular shaft and the handle is releasable.

15. Surgical instrument according to one of the preceding claims, wherein the actuating element (3) is mounted pivotably about a rotation point (5a), wherein the rotation point (5a) is arranged on the same side of the longitudinal axis (9) as the joint (12).

## Revendications

1. Instrument chirurgical comprenant :
une poignée (1a) comportant au moins un élément d'actionnement (3) ;
une tige tubulaire (2) reliée à la poignée et comportant un premier tube (7) et un deuxième tube (8) qui peut être déplacé le long de l'axe longitudinal (9) de la tige tubulaire (2) par rapport au premier tube (7) en actionnant l'élément d'actionnement (3) ; et
des premier et deuxième éléments formant mâchoire (10, 11) à l'extrémité distale de la tige tubulaire (2), le premier élément formant mâchoire (10) étant relié en position fixe à la tige tubulaire (2) et le deuxième élément formant mâchoire (11) étant relié de manière mobile à celle-ci par le biais d'une articulation (12) ;
le deuxième tube (8) étant relié à l'élément formant mâchoire mobile (11) par le biais d'un point de liaison (15b), de sorte que l'élément formant mâchoire mobile (11) peut pivoter autour de l'articulation (12) en actionnant l'élément d'actionnement (3) ;
**caractérisé en ce que** le point de liaison (15b) et une portion distale (11a) de l'élément formant mâchoire mobile (11), notamment l'extrémité distale de l'élément formant mâchoire mobile (11), sont disposés d'un côté de l'axe longitudinal (9) et l'articulation (12) du côté opposé de l'axe longitudinal (9), le point de liaison (15b) étant formé par une saillie (16) sur le côté extérieur de l'élément formant mâchoire mobile (11) qui se trouve d'un côté de l'axe longitudinal (9), laquelle peut être amenée en prise dans une cavité (14a) à l'extrémité distale du deuxième tube (8), et le deuxième tube (8) possédant à son extrémité distale une languette (14) qui s'étend dans la direction axiale, et la cavité (14a) étant disposée dans la languette (14).

2. Instrument chirurgical selon la revendication 1, **caractérisé en ce qu'**un élément jointif (15a) est formé entre l'extrémité distale du deuxième tube (8) et la cavité (14a), et **en ce qu'**une empreinte (17) est disposée sur le côté extérieur de l'élément formant mâchoire mobile (11), voisine de la saillie (16) dans la direction distale, l'élément jointif (15a) venant en prise dans l'empreinte (17) ou venant reposer dans celle-ci lorsque la saillie (16) sur l'élément formant mâchoire mobile (11) vient en prise dans la cavité (14a) dans le tube mobile.

3. Instrument chirurgical selon l'une des revendications précédentes, **caractérisé en ce que** l'extrémité distale du deuxième tube (8) possède une surface frontale (15) qui, lors d'un mouvement distal du deuxième tube (8) arrive contre une saillie supplémentaire (18) au niveau du point de liaison (15a) de l'élément formant mâchoire mobile (11) et ferme la partie formant bouche (4).

4. Instrument chirurgical selon l'une des revendications précédentes, **caractérisé en ce que** la saillie (16) est configurée montante dans la direction distale et/ou la languette est configurée flexible ou élastique.

5. Instrument chirurgical selon l'une des revendications précédentes, **caractérisé en ce que** le premier tube (7) est disposé à l'intérieur du deuxième tube (8).

6. Instrument chirurgical selon l'une des revendications précédentes, comprenant un dispositif de limitation entre le premier et le deuxième tube (7, 8) pour limiter le mouvement axial du deuxième tube (8) par rapport au premier tube (7), le dispositif de limitation comportant notamment une cavité (20) et une saillie (19), la saillie (19) étant maintenue dans la cavité en s'opposant à une force d'un ressort et étant mobile à l'intérieur de la cavité le long de l'axe longitudinal (9).

7. Instrument chirurgical selon la revendication 6, comprenant un mécanisme d'immobilisation (5), le mécanisme d'immobilisation (5), dans une première position, bloquant la saillie (19) à l'intérieur de la cavité (20) dans la direction radiale et, dans une deuxième position, autorisant un décalage radial de la saillie (19) de sorte que la saillie (19) puisse être déplacée hors de la cavité (20) contre la force du ressort.

8. Instrument chirurgical selon la revendication 7, le mécanisme d'immobilisation (5) comportant un anneau tournant (18a) et le mécanisme d'immobilisation pouvant être transféré de la première à la deuxième position en tournant l'anneau tournant, et la saillie (19) reposant notamment directement ou indirectement contre une paroi ou une surface, notamment un côté intérieur de l'anneau tournant, la paroi ou la surface présentant un écart radial de l'axe longitudinal qui varie le long du pourtour, et l'anneau tournant (18a) pouvant être déplacé par une torsion par rapport à la saillie, de sorte qu'une torsion de l'anneau tournant permet de modifier l'écart radial entre la saillie et l'axe longitudinal et de libérer la saillie hors de la cavité, ou le mécanisme d'immobilisation (5) possédant une languette élastique (25) qui, lors de l'actionnement d'un poussoir (22) monté sur ressort, se déplace dans le sens radial et pousse la saillie (19) hors de la cavité (20).

9. Instrument chirurgical (1) selon l'une des revendications précédentes, l'instrument (1) étant un instrument électrochirurgical bipolaire comportant une première et une deuxième électrode, la première électrode étant formée par l'élément formant mâchoire mobile (11) et la deuxième électrode étant disposée contre ou sur l'élément formant mâchoire fixe et étant isolée électriquement de celui-ci.

10. Instrument chirurgical (1) selon la revendication 9, **caractérisé en ce que** le premier et/ou le deuxième tube forment une connexion électrique entre la première électrode et une source de tension.

11. Instrument chirurgical (1) selon la revendication 9 ou 10, **caractérisé en ce que** la deuxième électrode est reliée à la source de tension par le biais d'un câble isolé électriquement vers l'extérieur, le câble s'étendant en direction de la poignée (1a) dans une rainure formée sur le côté extérieur du premier tube (7).

12. Instrument chirurgical (1) selon l'une des revendications précédentes, au moins le plus à l'extérieur des deux tubes (7, 8) possédant un gainage électriquement isolant.

13. Instrument chirurgical selon l'une des revendications précédentes, **caractérisé en ce que** des moyens d'actionnement supplémentaires, notamment une touche ou un poussoir, se trouvent sur la poignée (1a) pour la fermeture d'un circuit électrique.

14. Instrument chirurgical selon l'une des revendications précédentes, la liaison entre l'élément formant mâchoire mobile (11) et le deuxième tube (8) étant amovible au niveau du point de liaison (15b), et la liaison entre la tige tubulaire et la poignée étant amovible.

15. Instrument chirurgical selon l'une des revendications précédentes, l'élément d'actionnement (3) étant monté pivotant autour d'un point de rotation (5a), le point de rotation (5a) étant disposé du même côté de l'axe longitudinal (9) que l'articulation (12).
